# EUROPEAN PATENT SPECIFICATION

(11) **EP 0 638 316 B1**
(45) Date of publication and mention of the grant of the patent: **28.05.2003**
(21) Application number: 94305656.4
(22) Date of filing: 29.07.1994
(51) Int. Cl.: A61K 39/235, A61K 39/21, C12N 15/86

(54) **Recombinant adenovirus vaccines**
Rekombinante Adenovirus-Vakzinen
Vaccins à adénovirus recombinant

(30) Priority: 11.08.1993 US 105232; 20.07.1994 US 276289
(43) Date of publication of application: 15.02.1995
(73) Proprietor: Wyeth, Madison, New Jersey 07940-0874 (US)
(72) Inventor: Davis, Alan Robert,, Wayne, Pennsylvania, 19087 (US); Hung, Paul Porwen,, Bryn Mawr, Pennsylvania, 19010 (US); Lubeck, Michael David,, Glenmoore, Pennsylvania, 19343 (US); Natuk, Robert James,, Warrington, Pennsylvania, 18976 (US); Chanda, Pranab Kumar,, Wayne, Pennsylvania, 19087 (US); Murthy, Shridhara Chikkatur Shankaranarayana,, King of Prussia, Pennsylvania, 19406 (US); Lee, Shaw-Guang Lin,, Villanova, Pennsylvania, 19085 (US)
(74) Representative: Connelly, Michael John

(56) References cited:
- EP-A- 0 586 076
- WO-A-92/11028
- GB-A- 2 166 349
- US-A- 4 925 784
- CHEMICAL ABSTRACTS, vol. 121, no. 3, 1994, July 18, Columbus, Ohio, USA R.J. NATUK et al. "Immuno- genicity of recombinant human adenovirus-human immuno- deficiency virus vaccines in chimpanzees" pages 739-40, no. 32 713t; & AIDS Res. Hum. Retroviruses 1993, 9(5), 395-404
- NATUK, R.J., CHANDA P.K. ET AL: 'Adenovirus human immunodeficiency virus HIV envelope recombinant vaccines eleicit high titered HIV neutralizing anti....' PROC. NAT. ACAD. SCI. USA vol. 89, no. 16, 15 August 1992, pages 7777 - 7781
- NATUK R.J.: 'Immunogenecity of human adenovirus -human imunodeficiency virus vaccines in chimpanzees' AIDS RES HUM RETROVIRUSES vol. 9, no. 5, 1993, pages 395 - 404
- NATUK R. ET AL: ' Adenovirus vectored vaccines' DEVELOPMENTS IN BIOLOGICAL STANDARDIZATION vol. 82, 1994, BASEL, pages 71 - 77

## Description

### BACKGROUND OF THE INVENTION

A major goal of biomedical research is to provide protection against viral disease through immunization. One approach has been to use killed vaccines. However, large quantities of material are required for killed vaccine in order to retain sufficient antigenic mass. In addition, killed vaccines are often contaminated with undesirable products during their preparation. Heterologous live vaccines, using appropriately engineered adenovirus, which is itself a vaccine, seems like an excellent immunogen [Chanock R., JAMA, 195, 151 (1967)]. Our invention concerns vaccines using adenovirus as a vector.

Presently marketed adenovaccine comprises live, infectious adenoviruses in an enteric-coated dosage form. Upon administration to the patient to be vaccinated, the virus is carried past the upper-respiratory system (where disease-producing infection is thought to occur), and is released in the intestine. In the intestine, the virus reproduces in the gut wall, where, although it is not capable of causing adenoviral disease, nevertheless induces the formation of adenovirus antibodies, thus conferring immunity to adenoviral disease. In our invention, live, infectious adenovirus which has been engineered to contain genes coding for antigens produced by other disease-causing organisms. Upon release the virus will reproduce and separately express both the adenoviral antigen and the pathogen antigen, thereby inducing the formation of antibodies or induce cell mediated immunity to both adenovirus and the other disease-causing organism. By "live virus" is meant, in contradistinction to "killed" virus, a virus which is, either by itself or in conjunction with additional genetic material, capable of producing identical progeny. By "infectious" is meant having the capability to deliver the viral genome into cells.

Roy, in European Patent Publication 80,806 (1983), proposed a method for producing immunity to microbial diseases by the administration of a microbe containing a foreign gene which will express an antigen of a second microbe to which immunity is conferred. He states that preferred oral preparations are enteric coated. Dubelcco proposed recombinant adenovirus vaccines in which the surface protein of adenovirus is modified to contain in its structure a segment of foreign protein which will produce a desired biological response on administration to animals. [PCT International Publication WO 83/02393 (1983)]. Davis discloses oral vaccines derived from recombinant adenoviruses. [UK Patent GB 2166349 B].

Human immunodeficiency virus type 1 (HIV-1) has been etiologically associated with acquired immunodeficiency syndrome (AIDS) and related disorders. [Barre-Sinoussi, F., Science 220: 868 (1983); Gallo, R., Science 224: 500 (1984); Popovic, M., Science 224: 497 (1984); Sarngadharan, M., Science 224: 506 (1984)]. AIDS is now a worldwide epidemic for which, currently, there is no vaccine or cure. Most of the effort for vaccine development has focused on the envelope (env) glycoprotein as an antigen which might provide protective immunity. Antisera prepared against purified gp 120 can neutralize HIV-1 in vitro . [Crowl, R., Cell 41: 979 (1985); Putney, S., Science 234: 1392 (1986); Ho, D., J. Virol. 61: 2024 (1987); Nara, P., Proc. Natl. Acad. Sci. USA 84: 3797 (1987)]. HIV-1 envelope antigen has been produced in different expression systems including Escherichia coli [Crowl, R., Cell 41: 979 (1985); Chang, T., Bio/Technology 3: 905 (1985); Dawson, G., J. Infect. Dis. 157: 149 (1988)] as well as mammalian [Chakrabarti, S., Nature 320: 535 (1986); Dewar, R., J. Virol. 63: 129 (1989); Rekosh, D., Proc. Natl. Acad. Sci. USA 85: 334 (1988); Whealy, M., J. Virol. 62: 4185 (1988)] yeast [Barr, P., Vaccine 5: 90 (1987)] and insect cells [Hu, S., Nature 328: 721 (1978); Rusche, J., Proc. Natl. Acad. Sci. USA 84: 6294 (1987)].

Live recombinant vaccinia virus expressing the entire HIV-1 env glycoprotein [Hu, S., J. Virol. 61: 3617 (1987)] or purified recombinant gp 120 env glycoprotein [Berman, P., Proc. Natl. Acad. Sci. USA 85: 5200 (1988)] were evaluated in chimpanzees as vaccine candidates. Active immunization with these vaccines induced a good cell-mediated immune response as well as cytotoxic T-cell activity to the env antigen [Zarling, J., J. Immunol. 139: 988 (1987)]. All experimental animals seroconverted as assayed by ELISA and Western blotting. However, immunized chimpanzees developed no or only low titers of neutralizing antibody to HIV-1. Challenge with live virus failed to protect chimpanzees against these vaccines. Type-specific HIV-1 neutralizing antibodies were found in chimpanzees early in infection against a variable domain (V3) within the C-terminus half of gp 120 [Goudsmit, J., Proc. Natl. Acad. Sci. USA 85:4478 (1988)]. The recombinant gp 120 made in insect cells has also been shown to induce humoral immune response in goat (Rusche J., Proc. Natl. Acad. Sci. USA 84: 6294 (1987)]. Zagury [Nature 332: 728 (1988)] have demonstrated both anamnestic humoral and cellular immune reaction in humans using a vaccine virus recombinant expressing gp 160. [Chakrabarti, S., Nature 320: 535 (1986); Hahn, B., Proc. Natl. Acad. Sci. USA 82: 4813 (1985)]. Both group-specific cell-mediated immunity and cell-mediated cytotoxicity against infected T4 cells were also found. These results indicate that an immune state against HIV-1 can be obtained in humans using recombinant env-based vaccine. Recently, Desrosiers has shown that vaccination with inactivated whole simian immunodeficiency virus (SIV) can protect macaques against challenge with live SIV. [Proc. Natl. Acad. Sci. USA 86: 6353 (1989)]. These data provide hope that vaccine protection against human AIDS virus, HIV-1, infection may be possible.

Chanda discloses high level expression of the envelope glycoproteins of HIV-1 in the presence of rev gene using helper-independent adenovirus type 7 recombinants. [Virology 175: 535 (1990)]. Vernon discloses the ultrastructural characterization of HIV-1 gag subunit in a recombinant adenovirus vector system. [J. Gen. Virology 72: 1243 (1991)]. Vernon also discloses the preparation of the HIV-1 recombinant adenoviruses Ad7-rev-gag and Ad4-rev-gag.

### SUMMARY OF THE INVENTION

This invention provides a method of producing antibodies or cell mediated immunity to an infectious organism in a warm blooded mammal which comprises administering to said warm blooded mammal intranasally, intramuscularly, or subcutaneously, live recombinant adenoviruses in which the virion structural protein is unchanged from that in the native adenovirus from which the recombinant adenovirus is produced, and which contain the gene coding for the antigen corresponding to said antibodies or inducing said cell mediated immunity.

In its preferred embodiments, this invention provides a method of producing antibodies to human immunodeficiency virus (HIV-1), hepatitis B, hepatitis C, human papilloma virus, respiratory syncytial virus, rotavirus, or parainfluenza virus in a warm blooded mammal which comprises administering to said warm blooded mammal intranasally, intramuscularly, or subcutaneously, live recombinant adenoviruses in which the virion structural protein is unchanged from that in the native adenovirus from which the recombinant adenovirus is produced and which contain the gene coding for, respectively, human immunodeficiency virus, hepatitis B, hepatitis C, human papilloma virus, respiratory syncytial virus, rotavirus, or parainfluenza virus.

This invention also provides composition for producing antibodies or cell mediated immunity to an infectious organism in a warm blooded mammal, comprising live recombinant adenoviruses in which the virion structural protein is unchanged from that in the native adenovirus from which the recombinant adenovirus is produced, and which contain the gene coding for the antigen corresponding to said antibodies or inducing said cell mediated immunity, said composition being formulated in an intranasal, intramuscular, or subcutaneous dosage form.

Although this specification specifically refers to adenovirus of types 4, 5, or 7, live, infectious adenovirus of any type may be employed in this invention. Additionally, while the specification specifically refers to adenoviruses having an early region 3 (E3) deletion, adenoviruses which are attenuated, contain a temperature sensitive lesion, or a E1 deletion may also be used as a vector.

In a preferred embodiment, the method of treatment includes administering the recombinant adenovirus both prophylactically to an HIV-1 susceptible mammal and as immunotherapy following detection of HIV in said mammal. Regimens containing the following recombinant adenoviruses were used to produce the anti-HIV responses. These viruses were deposited under the terms of the Budapest Treaty with the American Type Culture Collection (ATCC) of 12301 Parklawn Drive, Rockville Maryland, 20852, USA, and were granted the accession numbers contained in the table below.

| Virus Name | Descriptive Name | ATCC Name |
|---|---|---|
| Ad7-env | Ad7-tplenv-tplHrev | VR-2299 |
| Ad7-gag | Ad7-tplgag-tplHrev | VR-2393 |
| Ad7-gag-1 | Ad7-rev-gag | VR-2392 |
| Ad4-env | Ad4-tplenv-tplHrev | VR-2293 |
| Ad4-gag | Ad4-tplgag-tplHrev | VR-2391 |
| Ad4-gag-1 | Ad4-rev-gag | VR-2390 |
| Ad5-env | Ad5-tplenv-tplHrev | VR-2297 |
| Ad5-gag | Ad5-tplgag-tplHrev | VR-2298 |
| Ad7-env_{MN} | Ad7-tplenv_{MN}-tplHrev | VR-2462 |
| Ad4-env_{MN} | Ad4-tplenv_{MN}-tplHrev | VR-2463 |
| Ad5-env_{MN} | Ad5-tplenv_{MN}-tplHrev | VR-2464 |

Referring to the above table Ad4, Ad5, and Ad7 refer to human adenoviruses types 4,5, and 7 respectively in which the E3 region has been deleted. Env refers to the HIV envelope glycoprotein (gp 160) gene. Gag refers to the HIV gag/pro gene. Rev refers to the HIV regulatory gene. Hrev refers to an altered version of the rev gene where the nucleotide sequences were changed without changing the amino acid sequence employing codons that were frequently used in human genes. Tpl refers to the upstream adenovirus tripartite leader sequence with an intervening sequence between the first and second leaders that are positioned in front of the recombinant genes. The constructs designated Ad7-env, Ad7-gag, Ad7-gag-1, Ad4-env, Ad4-gag, Ad4-gag-1, Ad5-env, and Ad5-gag contain either the gag or the env gene from the LAV strain of HIV and the constructs Ad7-env_{MN}, Ad4-env_{MN}, and Ad5-env_{MN} contain the env gene from the MN strain of HIV. The recombinant adenoviruses made from the LAV and MN strains of HIV-1 are illustrative of recombinant adenoviruses covered by this invention. This invention also covers recombinant adenoviruses which include the env and/or gag genes from other strains of HIV-1.

Both the Ad-env and Ad-env_{MN} adenoviruses were shown to replicate in human A549 cells and expressed recombinant env antigen in vitro demonstrating their capability of generating cell mediated, humoral, and secretory immunity in a mammal.

As described in detail below, intranasal administration of Ad-HIV recombinant viruses to naive chimpanzees resulted in both priming and boosting of both humoral and cell-mediated immune responses directed at HIV recombinant antigens. The recombinant adenoviruses administered to chimpanzees were shown to produce antibodies to the env and gag proteins of HIV. IgG antibodies specific for HIV were observed in nasal, saliva, and vaginal secretions following administration of the recombinant adenoviruses and IgA antibodies specific for HIV were observed in nasal and saliva secretions. The first set of recombinant viruses (Ad7) appeared to be shed the longest period of time and induce the best anti-Ad antibody response. The results also showed that administration of Ad-HIV vaccines by the intranasal route was superior to administration of enteric-coated recombinant viruses by the oral route.

Optimum immune responses directed at HIV antigens required primary infection one booster immunization with a heterotypic recombinant Ad-HIV to elicit strong anti-HIV binding antibodies. Intranasal administration of the Ad-HIV viruses effectively primed chimpanzees to respond with high titered neutralizing antibodies to HIV-1 following subsequent HIV-1 subunit protein booster immunization.

### BRIEF DESCRIPTION OF THE DRAWINGS

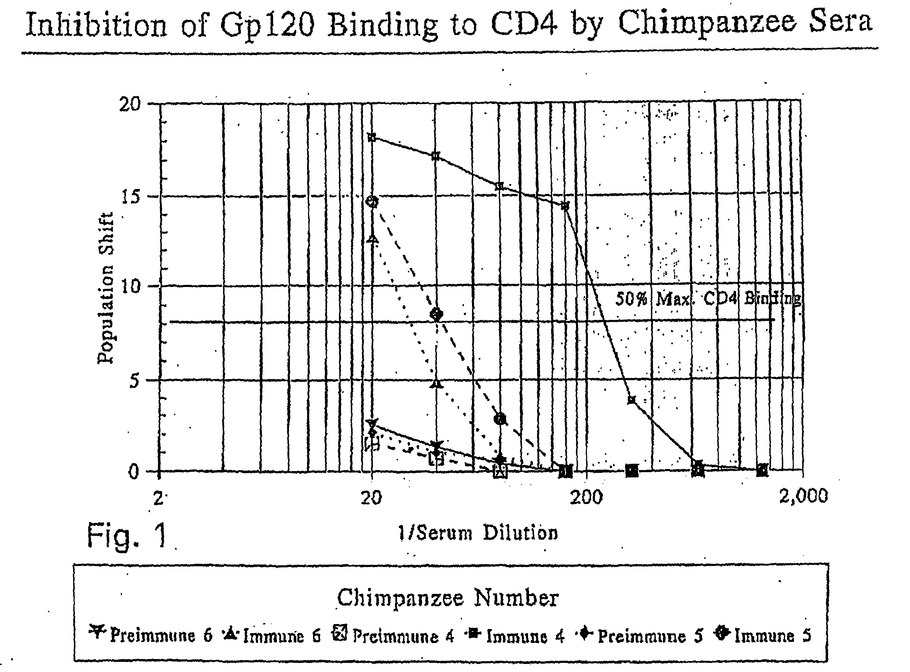

Figure 1 shows the inhibition of gp120 binding to CD4 by sera induced in dogs by recombinant Ad-HIV vaccines.

### DETAILED DESCRIPTION OF THE INVENTION

### Preparation of Representative Recombinant Adenoviruses

The following Examples show the construction of representative recombinant adenoviruses of this invention. The recombinant viruses were propagated on A549 cells and subsequently titered on A549 cells.

### Example 1. Ad7-gag-1

The construction of recombinant adenoviruses containing the gene for the HIV envelope protein has been described [Chanda, P., Virology 175: 535 (1990)]; a similar procedure was used to incorporate gag and pro [see Vernon, S., J. Gen. Virology 72: 1243 (1991)]. Briefly, a DNA fragment containing the entire gag and pro coding regions (bp 335 to 2165) of HIV-1 strain LAV [Wain-Hobson, S., Cell 40: 9, (1985)] was constructed with a unique Sa/I site in front of the AUG codon of the gag gene and an Xbal site at bp 2165, for the insertion of the viral rev-responsive element (rre; bp 7178 to 7698). A 2.37 kb SaI fragment containing the three HIV-1 sequences was inserted at a Sal site in an expression cassette containing the adenovirus type 7 (Ad7) major late promoter (MLP), the tripartite leader (TPL) with an intervening sequence between the first and second leaders, and the hexon polyadenylation site (poly A) as described in Chanda [Virology 175: 535 (1990)]. The cassette was inserted 159 bp from the right end of an Ad7 genome [Sussenbach, The Adenoviruses, Ginsberg, ed., Plenum Press, pp. 34-124 (1984)] containing the HIV-1 rev gene [Feinberg, M., Cell 46: 807 (1986); Sodroski, J., Nature 321: 412 (1986)] in a deleted [79.5 to 88.4 map units (m.u.)] E3 region [Chanda, P., Virology 175: 535 (1990)].

### Example 2. Ad4.gag-1

Following the procedure for the construction of the Ad7-gag-1 recombinant adenovirus in Example 1, a similar expression cassette containing analogous Ad4 sequences and the three HIV coding regions were inserted at a site 139 bp from the right end of an Ad4 genome which contained HIV-1 rev in an E3 deletion between 76 and 86 m.u.

### Example 3. Ad5-env

Ad5-tplenv-tplHrev contains the entire coding sequence of HIV-1 (LAV strain) gp160 and a modified version of the rev gene, called Hrev. Both the env as well as rev gene are preceded by a synthetic copy of the Ad5 tripartite leader (Ad5-tpl). Ad5-tpl was chemically synthesized and was cloned in pTZ vector. Then the gp160 DNA sequence was inserted behind the Ad5-tpl to create Ad5-tplenv/PTZ18R clone. The Hrev (∼360bp) was also chemically synthesized where the nucleotide sequences were changed without changing the amino acid sequence with the help of the codon usage. This was done to avoid homologous recombination as some identical sequences exist between env and rev. In an analogous way like Ad5-tplenv construct, Hrev gene was also inserted behind tpl in pTZ18R vector to create the plasmid, Ad5-tplHrev. The entire sequence containing Ad5-tplHrev was excised out and then inserted behind Ad5-tplenv to create the plasmid, Ad5-tplenv-tplHrev. This plasmid was then inserted in the deleted E3 region of Ad5 Marietta strain (78.8-85.7 mu deletion) at 78.8 mu. This plasmid was linearized with BglI enzyme and then mixed with 0-87 mu SnaB 1 fragment that was derived from the wild-type purified Ad5 virus. After A549 cells were transfected with the DNA mixtures, recombinant virus plaques were picked, plaque purified three times, and their genomic structures were confirmed by restriction endonuclease site analysis of DNA extracted from infected cells by the method of Hirt. [J. Mol Bio. 26: 365 (1967)].

### Example 4. Ad5-gag

Ad5-tplgag-tplHrev contains the entire gag and pro region as well as the modified rev gene, Hrev. A copy of the Ad5 synthetic tripartite leader was placed in front of the gag and Hrev genes. A DNA fragment containing the entire gag and pro regions (bp 335 to 2165 of LAV strain of HIV-1) was constructed with a unique SalI site in front of AUG codon of the gag gene and an xba site at bp 2165, for the insertion of the viral rev-responsive element (rre; bp 7178-7698). Two separate plasmids Ad5-tplgag as well as Ad5-tplHrev were constructed in a similar way as described for Ad5-tplenv-tplHrev. Then the Ad5-tplHrev fragment was inserted behind Ad5-tplgag to create the plasmid Ad5-tplgag-tplHrev. Then the fragment Ad5-tplgag-tplHrev was inserted at the unique XbaI site at map position 78.8 of the Ad5 Marietta strain with an E3 deletion (78.8-85.7 mu E3 deletion). Then the final plasmid containing the Ad5 sequence was linearized and then mixed with the 0-87 mu SnaB1 viral fragment for transfection. Recombinant plaques were picked up, plaque purified three times, and were checked by Hirt analysis of DNA extracted from the infected cells.

### Example 5. Enteric Coated Capsules

Recombinant adenoviruses were grown in A549 cells and harvested following 3 cycles of freeze-thawing. Clarified infected cell lysates were lypholized and 60 to 100 mg were packed into #2 gelatin capsules using a 1 ml syringe plunger under dehumidified conditions. The capsules were coated with a 10% cellulose acetate phthalate in acetone/100% Ethanol (1:1) by manually dipping each end 6 times with air drying between dips. A coating between 69 to 77 mg of cellulose acetate phthalate was formed under these conditions. Sample capsules were tested for resistance to simulated gastric fluid (0.32% pepsin, 0.2% NaCI pH 1.2) at 37 C using a VanKel Disintegration Testor apparatus for 1 hr. The capsules were inspected for holes or cracks and transferred to a 15 ml tube containing 10 ml of simulated intestinal fluid (1.0% pancreatin, 0.05 M monobasic potassium phosphate pH 7.5) and rotated at 37° C. All capsules tested were resistant to simulated gastric fluid for 1 hr at 37 C with agitation and began to dissolve within 15 min. in simulated intestinal fluid. The amount of virus was titrated on confluent A549 cell monolayers by a plaque assay and the viral DNA stability confirmed by Hirt analysis.

### Example 6. Ad7-env_{MN}

The construction of recombinant adenoviruses containing the coding sequence of the env (gp 160) gene of MN strain of HIV-1 is described briefly as follows: The 125 bp (6243 to 6367) fragment of the amino (NH₂) terminus of the env (gp160) gene including the initiation codon (ATG) as well as consensus Kozak sequence was amplified by polymerase chain reaction (PCR) from the clone pMNST 1-8-9. This fragment was then cloned in pGEM vector and the resultant clone was designated as pGEMMNenv. The following fragments of DNA were isolated by digesting with the restriction enzymes KpnI and XbaI from the clone PAd5tpl_{MN}env 223 (6367 bp to 8816 bp), XhoI + KpnI fragment from PGEMenv and salI + XbaI fragment from pAd7tpl 18RD. All of these fragments were ligated together and the resultant clone was designated as pAd7tpl_{MN}env. This plasmid was then digested with XbaI and treated with calf intestine alkaline phosphatase (CIAP). The NheI+XbaI fragment of Hrev gene was then isolated from the plasmid, pAd7tplHrev 18RD. The clone that was obtained after ligating these two fragments together was designated as pAD7tpl_{MN}envtplHrev. This plasmid was then digested with NheI+ Xbal and then ligated to the E3 deletion plasmid of Ad7, pAd7ΔE3 (68 m.u. to 100 m.u. deletion) that was also digested with XbaI and then treated with CIAP. The resultant plasmid was designated as pAD7ΔE3tpl_{MN}envtpl_{MN}Hrev. This plasmid was digested with EcoRI and mixed with the EcoRI (0-87 m.u.) fragment of the Ad7 genomic DNA. A549 cells were then transfected with these DNAs. Recombinant plaques obtained from in vivo recombination were identified by the appropriate restriction digestion analyses of the Hirt DNA. The plaques were also identified by the production of gp1601 gp120, and gp41 using appropriate antibodies on Western blots.

### Example 7. Ad4-env_{MN} and Ad5-env_{MN}

The construction of Ad4 and Ad5 recombinants are the same as that of Ad7-env_{MN} except that for Ad4, EcoRI digested DNA from pAd4ΔE3tpl_{MN}envtplHrev was combined with the Bell (0-87 m.u.) fragment from the Ad4 genomic DNA to produce the recombinant Ad4 virus. Similarly for Ad5, MluI-digested DNA from pAd5ΔE3tpl_{MN}envtplHrev was combined with the SpeI (0-75 m.u.) fragment of Ad5 genomic DNA to produce the recombinant Ad5 adenovirus. Like Ad7, both Ad4 and Ad5 recombinants were obtained from A549 cells.

### Example 8. Subunit Antigen Preparation

gp-120_{MN} was prepared according to Kaufman, R.J., Nucleic Acid Res. 19: 4485 (1991) and was used in SAF-m adjuvant (Allison, A.C., J. Imm. Meth. 95: 157 (1986). gp-120_{SF2} was prepared according to Scandella, C.J., AIDS Res. Human Retroviruses 9: 1233 (1993) and was used in MF-59 adjuvant (Keitel, W., Vaccine 11: 909 (1993)). HA-env_{K17K} was prepared according to Kalayan, N., Vaccine 12: 753 (1994) and was used in SAF-m adjuvant.

### Measurement of Replication and Antigen Expression

Human A549 cells were infected (MOI 10:1) with recombinant adenovirus types 4, 5, and 7 that contained either the LAV or MN env genes. At 34 hours post-infection, virus titer and env antigen expression was determined in duplicate samples. One dish of infected cells was subjected to 3 cycles of freeze thawing and the cell lysate was tested for the presence of infectious virus by plaque assay. The second culture dish was washed, detergent solubilized, and an aliquot of the cell lysate was loaded on to a 10% polyacrylamide gel. Following electrophoresis, the separated proteins were transferred to nitrocellulose by a Western blot apparatus. The transferred proteins were immunostained with anti-env reagents. A known standard, recombinant gp160, was added prior to electrophoresis. The resulting immunoblot was scanned by a densitometer and the amount of recombinant env determined. There were no significant differences seen between wild type adenoviruses and the recombinant adenoviruses expressing either the LAV or MN env gene. Both types of recombinant adenoviruses, LAV or MN, produced similar amounts of env antigen. Therefore, both types of Ad-env recombinants, LAV and MN, were able to grow in human A549 cells as well as their corresponding wild type adenovirus, and were able to express recombinant env antigen. These results therefore demonstrate that both the LAV and MN adenovirus recombinants are capable of generating cell mediated, humoral, and secretory immunity in a mammal. The data obtained are summarized in the table below.

| ADENOVIRUS REPLICATION AND ANTIGEN EXPRESSION | | |
|---|---|---|
| Adenovirus | pfu/cell x 10² | µg env/10⁶ cells |
| Ad4 wild type | 5.4 | 0 |
| Ad4-env | 9.1 | 2.1 |
| Ad4-env_{MN} | 6.8 | 2.7 |
| | | |
| Ad5-wild type | 22 | 0 |
| Ad5-env | 86 | 5.4 |
| Ad5-env_{MN} | 18 | 5.7 |
| | | |
| Ad7-wild type | 18 | 0 |
| Ad7-env | 11 | 3.1 |
| Ad7-env_{MN} | 7.8 | 3.6 |

### Treatment Regimens

Immunogenicity of the recombinant adenoviruses for HIV was evaluated in chimpanzees under four treatment regimens (1,2, 3, and 6), and in dogs two treatment regimens (4 and 5). Protection against HIV-1 infection was evaluated in chimpanzees in the sixth treatment regimen. The first regimen consisted of administering the recombinant adenovirus orally via an enterically coated capsule (Example 5) at 0,7, and 26 weeks followed by an env + gag subunit protein booster using alum as an adjuvant. The second regimen consisted of further treating the chimpanzees that received regimen 1 at 46 and 58 weeks with additional boosters of recombinant adenovirus administered intranasally. The third treatment regimen consisted of administering recombinant adenovirus intranasally to naive chimpanzees at weeks 0, 24, and 52 followed by an env subunit booster at week 75. The fourth treatment regimen consisted of administering recombinant adenoviruses derived from both the LAV and MN strains of HIV-1 to dogs.

The fifth treatment regiment consisted of administering env subunit boosters to either previously immunized or control dogs. Each treatment group consisted of 6 previously immunized dogs and 2 control dogs. Of the previously immunized dogs, six had received Treatment Regimen 4 (Group A); six had received Treatment Regimen 4 (Group D); six had received Ad-env_{HXB2} (expressing a portion of the HIV env V3 loop, derived from the LAV strain of HIV); and twelve had previously received Ad-env_{HXB2} (expressing a portion of the HIV env V3 loop, derived from the MN strain of HIV) (prepared according to Robert-Guroff, M., J. Virol 68: 3459 (1994) and Veronese, F.D., J. Biol. Chem. 268: 25894 (1993)).

The sixth treatment regimen consisted of administering Ad-env_{MN} recombinants to chimpanzees, followed by 0, 1 or 2 Ad-env_{MN} booster immunizations using heterologous Ad vectors. The chimpanzees were then given one or two booster immunizations with env (gp120_{SF2}) subunit antigen preparations, followed by a challenge with the SF2 strain of HIV.

The following table summarizes treatment regimens 1 and 2.

| **TREATMENT REGIMENS 1 AND 2** | | | |
|---|---|---|---|
| **Immunization** | **Time** | **Chimpanzees 1 and 2** | **Chimpanzee 3** |
| **Regimen 1** | | | |
| Primary* | 0 weeks | 1.5 x 10⁷ pfu Ad7-env 2.0 x 10⁹ pfu Ad7-gag-1 | 1.5 x 10⁷ pfu Ad7-env |
| | | | |
| 1st Booster* | 7 weeks | 1.1 x 10¹⁰ pfu Ad4-env 1.0 x 10¹⁰ pfu Ad4-gag-1 | 1.1 x 10¹⁰ pfu Ad4-env |
| | | | |
| 2^{nd} Booster* | 26 weeks | 7.9 x ¹⁰ pfu Ad5-env | 7.9 x 10¹⁰ pfu Ad5-env |
| | | | |
| 3rd Booster⁺ | 34 weeks | 200 ug env in 0.2% alum 500 ug env in 0.2% alum | 200 µg env in 0.2% alum |

| **Regimen 2** | | | |
|---|---|---|---|
| 1st Intranasal Boost | 46 weeks | 1.0 x 10⁸ pfu Ad7-env 1.0 x 10⁸ pfu Ad7-gag | 1.0 x 10⁸ pfu Ad7-env |
| | | | |
| 2nd Intranasal Boost | 58 weeks | 1.0 x 10⁸ pfu Ad4-env 1.0 x 10⁸ pfu Ad4-gag | 1.0 x 10⁸ pfu Ad4-env |

| | | | |
|---|---|---|---|
| *Each dose was administered in enteric-coated gelatin capsules on 3 consecutive days. | | | |
| ⁺Administered intramuscularly. | | | |

The following table summarizes treatment regimen 3.

| **TREATMENT REGIMEN 3** | | | |
|---|---|---|---|
| **Immunization** | **Time** | **Chimpanzees 4 and 5** | **Chimpanzee 6** |
| Primary* | 0 weeks | 1.0 x 10⁷ pfu Ad7-env 1.0 x 10⁷ pfu Ad7-gag | 1.5 x 10⁷ pfu Ad7-env |
| | | | |
| 1st Booster* | 24 weeks | 1.0 x 10⁷ pfu Ad4-env 1.0 x 10⁷ pfu Ad4-gag | 1.5 x 10⁷ pfu Ad4-env |
| | | | |
| 2nd Booster* | 52 weeks | 1.0 x 10⁷ pfu Ad5-env 1.0 x 10⁷ pfu Ad5-gag | 1.5 x 10⁷ pfu Ad5-env |
| | | | |
| 3rd Booster⁺ | 75 weeks | 0.5 mg env | 0.5 mg env |

| | | | |
|---|---|---|---|
| *Administered intranasally. | | | |
| ⁺Administered intramuscularly. | | | |

The following Table summarizes treatment regimen 4.

| **TREATMENT REGIMEN 4** | | | | | |
|---|---|---|---|---|---|
| | | **Group A** | **Group B** | **Group C** | **Group D** |
| **Immunization** | **Time** | **(n=6)** | **(n=3)** | **(n=3)** | **(n=6)** |
| Primary* | 0 weeks | Ad7-env_{MN} | Ad7-env | Ad7-env_{MN} + Ad7-env | Ad5-env_{MN} |
| 1st Booster* | 12 weeks | Ad5-env_{MN} | Ad5-env | Ad5-env_{MN} +Ad5-env | Ad4-env_{MN} |

| | | | | | |
|---|---|---|---|---|---|
| * Each recombinant adenovirus was administered intratracheally at a dose of 1 x 10⁹ per dog. | | | | | |

The following summarizes treatment regimen 5. Each group consisted of 6 dogs that were previously immunized, as described above, and 2 control dogs. Each group received 50 µg of the subunit in adjuvant at 0 weeks (20-28 weeks from the last Ad-env administration). Group A received gp120_{SF2} in MF59 adjuvant; Group B received CHO-derived gp120_{MN} (antibody purified) in SAF-m; Group C received Ad5-gp160_{MN}-derived gp160_{MN} (lentil lectin purified) in SAF-m; Group D received Ad5-gp160_{MN}-derived gp160_{MN} (lentil lectin purified) in MF59; and Group E received HA-env_{K17K} (expressing a portion of the HIV env V3 loop). Twelve weeks later dogs were identically boosted with the same subunit, with the exception of Group D dogs which were reboosted with the HA-env_{K17K}.

The following table summarizes treatment regimen 6.

| **TREATMENT REGIMEN 6** | | | | | | |
|---|---|---|---|---|---|---|
| Immunization | Time (weeks) | Chimpanzee Number | | | | |
| | | 7 | 8 & 9 | 10 | 11 | 12 |
| Primary | 0 | Ad5-env_{MN}⁺ | Ad5-env_{MN} | Ad5-env_{MN}, Ad7-env_{MN}, Ad4-env_{MN} | Ad5-env_{MN} | Ad5 wild type |
| 1st Booster | 12 | - | Ad7-env_{MN} | Ad5-env_{MN}, Ad7-env_{MN}, Ad4-env_{MN} | Ad7-env_{MN} | Ad7 wild type |
| 2nd Booster | 24 | - | - | - | Ad4-env_{MN} | Ad4 wild type |
| Subunit Boost | 26 | gp120_{SF2}* | - | - | - | - |
| Subunit Boost | 38 | gp120_{SF2} | gp120_{SF2} | gp120_{SF2} | - | |
| Subunit Boost | 48 | - | - | - | gp120_{SF2} | MF59 |
| Challenge | # | HIV_{SF2} | HIV_{SF2} | | HIV_{SF2} | HIV_{SF2} |

| | | | | | | |
|---|---|---|---|---|---|---|
| ⁺ All Ad-env and Ad viruses were administered at a dose of 1.0 x 10⁷ pfu/virus intranasally. | | | | | | |
| * 50 µg HIV gp120_{SF2} formulated in MF59 adjuvant was administered intramuscularly. | | | | | | |
| # Chimpanzees 7,8, and 9 were challenged at 40 weeks; 11 and 12 were challenged at 52 weeks, and 10 was not challenged. | | | | | | |

### Measurement of Immunogenicity: Treatment Regimen 1 Chimpanzee Inoculations

Three chimpanzees (2 males and 1 female) that were screened negative for the presence of neutralizing antibodies to human adenoviruses type 4, and 7 were evaluated using treatment regimen 1. Enteric-coated capsules containing recombinant adenoviruses were given using a stomach tube under anesthesia on three consecutive days. Two chimpanzees (1 and 2) received both env and gag recombinant viruses while the third chimp (3) received only env recombinant viruses.

Adenovirus-derived subunit preparations containing env or gag gene products were purified from infected A549 cell cultures [see Vernon, S., J. Gen. Virology 72: 1243 (1991) and Natuk, R., Proc. Natl. Acad. Sci. USA 89: 7777 (1992)]. Recombinant antigens were formulated with alum adjuvant and administered intramuscularly, 200 ug/dose env and 500 ug/dose gag particles.

Whole blood, serum, and stool samples were collected at different times during the course of the experiment. Whole blood was processed to obtain white blood cell populations for FACS, HIV CTL (using recombinant vaccinia viruses expressing HIV-env, HIV-gag, or the lac gene products), and for lymphoproliferative assays to purified HIV recombinant gp160, gp120, and p24. Serum and stool specimens were stored at -70° C until use.

### Detection of Recombinant Adenoviruses in Stool Specimens

Chimpanzee stool specimens were thawed and 10% (V/V) suspensions were made into antibiotic containing DMEM. Clarified stool suspensions were used to infect confluent A549 cell monolayers in 60 mm tissue culture dishes. After a 1 hr adsorption period the unbound material was washed away and the monolayers were overlaid with an 0.5% agar overlay medium. Plaques were allowed to develop for 7-10 days and plaques were visualized by neutral red staining, counted and the agar overlay was gently removed taking care not to disturb the cell monolayer. The cell sheet was transferred to nitrocellulose filter membranes (Millipore Type HA, 0.45 um), presoaked in 20X SSC and placed on the cell layer and left in contact with the cell monolayer for 2 to 4 minutes. The filters were peeled off, air-dried, and baked for 2 hr in a vacuum oven at 80° C. Nitrocellulose filters were washed twice in 3X SSc/0.1% SDS at room temperature and prehybridized and hybridized according to standard procedures [Poncet, D., J. Virol. Methods 26: 27 (1989)]. ³²P-labeled oligo-probes were added to the hybridization buffer (1 x 10⁶ CPM) and incubated overnight at 42° C. DNA probes were prepared by which could detect either Ad4 fiber, Ad5 fiber, Ad7 fiber, HIV-env or HIV-gag specific sequences. [Wain-Hobson, Cell 40: 9 (1985)]. The filters were washed, autoradiographed, and hybridization signals were counted.

### Adenovirus Neutralization Test Procedures

Serial 2-fold dilutions (starting with 1:4) of heat-inactivated (56 C fdor 30 min.) dog serum were made in 96-well microtiter plates (0.05 ml/well) and were mixed with 0.05 ml media containing 30-100 TCID₅₀ virus for 1 hr at 37° C. To each well 0.05 ml of media containing 2x 10⁴ A549 cells were added and the plates were incubated at 37° C 5% CO₂ for 7-10 days. All samples were done in duplicate. Virus and uninfected cell controls were included in each assay for determining the end point in test sera. Titers were expressed as the reciprocal of the lowest dilution at which 50% cytopathic effect was observed.

### Detection of Anti-HIV Antibodies by ELISA and Western Blotting

Detection of anti-HIV antibodies Chimpanzee antibody responses to HIV-1 antigens were measured by testing various dilutions by commercial ELISA and Western blot kits as instructed by the manufacturers (DuPont, Wilmington, DE).

### Results

Feces were collected from each chimpanzee prior to and after virus inoculation and stored at -70° C. Ten percent suspensions were prepared from each sample and were used to infect confluent A549 cell monolayers. After 7-10 days viral plaques were identified by neutral red staining and the cell monolayers were transferred to nitrocellulose membranes. Representative samples were hybridized with various labeled oligo-probes to detect sequences specific for Ad4, Ad5, Ad7, HIV-env, or HIV-gag genes. Identification of specific recombinant Ad-HIV viruses could be determined by this plaque hybridization technique. None of the recombinant viruses were shed into the feces for longer than 7 days p.i. Peak titers were always associated with 1-3 day samples and most likely represented the non-adsorbed virus inoculum. Previous chimp studies using Ad-HBsAg recombinants had indicated that Ad-HBsAg recombinants could be detected for 30-40 days p.i. With the enteric capsule route of administration, it appeared that these recombinant viruses did not replicate well in vivo.

Seroconversion to the serotype of the adenovirus vectors employed was determined by neutralization test procedures. Very low to modest anti-adenovirus serum titers were measured to all 3 serotypes used in each of the chimpanzees.

Seroconversion to recombinant HIV gene products were determined by either ELISA or Western blotting techniques. No ELISA response was detected in any of the chimpanzees prior to the second booster inoculation with the Ad5-env recombinant. Two weeks following Ad5-env inoculation anti-env responses could be measured in 2 of the 3 animals. Intramuscular injection of gag and/or env preparations had a slight boosting effect in 1 of the 3 animals. Western blot analysis appeared to be much more sensitive than the ELISA and had the further advantage of identification of which env and/or gag gene products were being recognized as being immunogenic. Low serum antibody titers were measured following both the primary Ad7 recombinant and first booster with Ad4 recombinants viruses. A significant increase in serum titer to env gene products was observed following the second booster immunization with the Ad5-env recombinant. Significant increases in the 2 animals which received gag gene products were seen following injection with subunit preparations. Despite relatively good Western blot titers to HIV antigens, only 1 of the 3 animals responded with serum neutralizing antibodies. This response in chimpanzee 2 was very low (titer of 10 to 20).

These results are summarized in the following table.

| **RESULTS OBTAINED USING TREATMENT REGIMEN 1** | | | | | | |
|---|---|---|---|---|---|---|
| Chimp Number | Recombinant Virus | Recombinant Virus Shedding Stools (Days) | Peak Anti-Adeno Neutralizing Titer | Western Blot Peak anti-HIV Titers | | Peak Anti-HIV Neutralizing Titer |
| | | | | env | gag | |
| 1 | Ad7-env,Ad7-gag-1 | 2,2 | 128 | - | 20 | <10 |
| | Ad4-env,Ad4-gag-1 | 2,2 | 8 | - | 20 | <10 |
| | Ad5-env | 7+ | 128 | 100 | - | <10 |
| | subunit: env + gag | | | 100 | 1000 | <10 |
| | | | | | | |
| 2 | Ad7-env, Ad7-gag-1 | 3,2 | 64 | - | 20 | <10 |
| | Ad4-env, Ad4-gag-1 | 1,7 | 128 | 20 | 100 | <10 |
| | Ad5-env | 7+ | 64 | 10000 | - | 20 |
| | subunit: env + gag | | | 1000 | 10000 | 10 |
| | | | | | | |
| 3 | Ad7-env | 2 | 6 | 20 | N/A* | <10 |
| | Ad4-env | 1 | 128 | 20 | N/A | <10 |
| | Ad5-env | 7+ | 512 | 1000 | N/A | <10 |
| | subunit: env | | | 100 | N/A | <10 |

| | | | | | | |
|---|---|---|---|---|---|---|
| *N/A = not applicable. | | | | | | |

Cell-mediated immunity was measured in peripheral blood mononuclear cell population obtained from chimpanzees. HIV specific CTL activity was measured by determining lysis of syngenic target cells that were infected with vaccinia virus recombinants that express either the HIV-env gene products, the HIV-gag gene products, or the lac gene product (control for nonspecific cytotoxicity). A hint of HIV specific CTL-like activity was measured in this way.

Lymphoproliferative assays were performed to determine whether purified recombinant env (gp160, gp120) or gag (p24) preparations were capable of stimulating blastogenesis. No proliferation was measured after the primary inoculum and only 1 of the 3 animals show a lymphoproliferative response following administration of the first boost with Ad4 recombinant viruses. All 3 animals responded with proliferative responses after the second booster (Ad5-env) and the third boost (subunit preparations).

### Measurement of Immunogenicity: Treatment Regimen 2 Chimpanzee Inoculations and Collection of Data

Three chimpanzees (2 males and 1 female) that were previously inoculated with Ad-HIV recombinant viruses in enteric-coated capsules and boosted with adenovirus-derived gag and/or env subunits (treatment regimen 1) were infected intranasally with Ad7-HIV viruses (week 46) and 12 weeks later (week 58) with Ad4-HIV viruses. Recombinant adenoviruses were given in tissue culture media diluted with phosphate saline buffer dropwise into the nostrils of chimpanzees under anesthesia. Two chimpanzees (numbers 1 and 2) received both env and gag recombinant viruses while the third chimp (number 3) received only env recombinant viruses.

Whole blood, serum, and stool samples were collected at different times during the course of the experiment, and processed as described in Regimen 1. Adenovirus detection in stool samples or nasal swabs, adenovirus neutralization test procedures, and detection of anti-HIV antibodies were performed according to the procedures described in Regimen 1.

### Results

The first intranasal booster with Ad7 recombinants was given in one dose of 1x10⁸ pfu's/chimpanzee. At the time of virus administration chimpanzees 3, 1, and 2 had serum anti-Ad7 neutralization titers of <4, 8, and 64 respectively from previous oral immunizations. Nasal swabs and stool samples were examined for the presence of shed recombinant viruses by a plaque hybridization technique. Recombinant Ad7-env was detected in nasal swabs up to 7 days p.i. in two of the animals. Recombinant Ad7-env and Ad7-gag were found to be present in stool samples from 5 to 12 days p.i. There was a correlation between the serum titer to Ad7 and the ability to detect recombinant viruses in nasal swabs and stool specimens. The two animals which displayed marginal anti-HIV antibody response were greatly augmented by the intranasal boost. The third animal was boosted to a lesser extent. Low titered neutralizing antibodies directed at HIV could now be detected in all three animals. Secretory antibodies were detected in nasal swab specimens which contained anti-gag and/or env binding antibodies. No signs or symptoms of respiratory disease were observed in these animals as a result of intranasal administration of the Ad7 recombinant viruses.

Three months later these chimpanzees were immunized with Ad4 recombinants at a single dose of 1x10⁸ pfu's/chimpanzee/virus. These animals had serum anti-Ad4 neutralization titers between 128 to 256 from previous oral immunization at the time of intranasal challenge. At 3 days post-infection 2 of the animals (2 and 3) had a slight cough. The third animal (number 1) died on day 5 from a bacterial pneumonia (Streptococcus pneumoniae was isolated). The other two animals presented harsh sounds by auscultation and S. pneumoniae was isolated from both chimpanzees. Antibiotic treatments were initiated and both chimpanzees recovered.

Upon retrospective examination of this situation several observations could be made. At the time of intranasal administration chimpanzee number 1 was already experiencing a fever and an abnormal Complete Blood Count. There was a disproportionate number of polymorphonuclear cells present and a 5% level of band cells (immature polymorphonuclear cells) taken together, this information indicated that there was a significant bacterial infection taking place prior to virus inoculation. Autopsy specimens taken from the lung, liver, spleen, and serum all tested negative for the presence of infectious adenovirus by tissue culture using 3 blind passages on susceptible A549 cell monolayers. Similar findings were obtained by plaque hybridization techniques. Lung and liver paraffin embedded samples tested negative for the presence of adenovirus antigens using a commercial immunofluorescent kit for adenovirus antigens. Inclusion bodies were observed in H&E stained lung sections. There was a disagreement by experts as to whether these inclusions were caused by adenovirus or not. Several weeks later another chimpanzee experienced a similar fate at the same primate center and died. While it was likely that administration of recombinant adenoviruses had a only a minor role, if any, in causing the death of chimpanzee number 1 it was considered prudent to administer antibiotics prophylactically prior to and after any further intranasal administration of adenovirus recombinants to chimpanzees.

The following table shows the results obtained using treatment Regimen 1 and the Ad7-recombinants in Regimen 2.

| **RESULTS OBTAINED USING TREATMENT REGIMENS 1 AND 2** | | | | | | |
|---|---|---|---|---|---|---|
| Chimp Number | Recombinant Virus | Recombinant Virus Shedding Stools (Days) | Peak Anti-Adeno Neutralizing Titer | Western Blot Peak anti-HTV Titers | | Peak Anti-HIV Neutralizing Titer |
| | | | | env | gag | |
| 1 | **Regimen 1** | | | | | |
| | Ad7-env, Ad7-gag-1 | 2,2 | 128 | - | 20 | <10 |
| | Ad4-env,Ad4-gag-1 | 2,2 | 8 | - | 20 | <10 |
| | Ad5-env | 7+ | 128 | 100 | - | <10 |
| | subunit: env + gag | | | 100 | 1000 | <10 |
| | **Regimen 2** | | | | | |
| | Ad7-env,Ad7-gag | 12 | 512 | 10000 | 10000 | 10 |
| | | | | | | |
| 2 | **Regimen 1** | | | | | |
| | Ad7-env, Ad7-gag-1 | 3,2 | 64 | - | 20 | <10 |
| | Ad4-env,Ad4-gag-1 | 1,7 | 128 | 20 | 100 | <10 |
| | Ad5-env | 7+ | 64 | 10000 | - | 20 |
| | subunit: env + gag | | | 1000 | 10000 | 10 |
| | **Regimen 2** | | | | | |
| | Ad7-env, Ad7-gag | 9 | 8192 | 1000 | 10000 | 20 |
| | | | | | | |
| 3 | **Regimen 1** | | | | | |
| | Ad7-env | 2 | 6 | 20 | N/A* | <10 |
| | Ad4-env | 1 | 128 | 20 | N/A | <10 |
| | Ad5-env | 7+ | 512 | 1000 | N/A | <10 |
| | subunit: env | | | 100 | N/A | <10 |
| | **Regimen 2** | | | | | |
| | Ad7-env | 7 | 256 | 10000 | N/A | 10 |

| | | | | | | |
|---|---|---|---|---|---|---|
| *N/A = not applicable. | | | | | | |

### Measurement of Immunogenicity: Treatment Regimen 3 Chimpanzee Inoculations and Collection of Data

Three chimpanzees (2 males and 1 female) that were screened negative for the presence of neutralizing antibodies to human adenoviruses type 4, 5, and 7 were evaluated using treatment regimen 3. Two chimpanzees (numbers 4 and 5) received both env and gag recombinant viruses while the third chimp (number 6) received only env recombinant viruses. Antibiotics were administered prophylactically to the chimpanzees and no respiratory disorders were observed.

Whole blood, serum, and stool samples were collected at different times during the course of the experiment, and processed as described in Regimen 1. Adenovirus detection in stool samples or nasal swabs, adenovirus neutralization assays, and detection of anti-HIV antibodies were performed according to the procedures described in Regimen 1.

### Detection of Inhibition of Gp120 Binding to CD4 Binding Sites

This assay is designed to measure the ability of chimpanzee anti-env antibodies to block the interaction of the HIV gp120 antigen with in natural ligand CD4. Various dilutions of chimpanzee sera were incubated with purified recombinant gp120 (1 ug/ml) 37°C for 1 hour. HeLa CD4 positive cells (5 x 10⁵) were added to this mixture and incubated at 4°C for 1 hour. The cells were washed 3 times with PBS-5%BSA and mixed with a FITC-labeled monoclonal antibody directed at the CD4 antigen (same site the gp120 binds to) and incubated at 4°C for 1 hour. The cells were washed three times with the PBS-5% BSA and analyzed by flow cytometry.

### Results

**1st Immunization with Ad7-recombinants:** Recombinant viruses were shed into feces for 22 to 34 days post-infection. No recombinant viruses were detected in nasal secretions taken at 2 weeks post-infection. Seroconversion to the serotype of the adenovirus vectors employed was determined by neutralization assays. Excellent anti-adenovirus serum titers were measured in all 3 chimpanzees to Ad7 serotypes used in each of the chimpanzees. Seroconversion to recombinant HIV gene products were determined by Western blotting. Four weeks following the primary immunization with Ad7-recombinants anti-env and anti-gag responses could be measured in 2 of the 3 chimpanzees. By 20 weeks post-infection all 3 animals had measurable antibodies to HIV antigens. Secretory antibodies were not found in nasal swabs taken within the first 4 weeks following primary immunization. All 3 chimpanzees failed to mount detectable anti-HIV neutralizing antibody responses.

**1st Booster Immunization with Ad4-recombinants:** Recombinant Ad4 viruses were shed into feces for 14-28 days post-infection. Examination of nasal swabs indicated that recombinant Ad4 viruses could be detected in all 3 chimpanzees for at least 7 days post-infection. Significant anti-Ad4 responses were mounted against the Ad4 serotype following intranasal administration . The magnitude was slightly lower then that measured against the Ad7-recombinant viruses. Excellent booster responses to gag and/or env antigens were measured in all three animals. Low titered (1:2) anti-gag and/or anti-env responses were measured in nasal swabs from Chimpanzees 4 and 5. Still no anti-HIV neutralizing antibodies were measured in any of the animals.

**2nd Booster Immunization with Ad5-recombinants:** Recombinant Ad5 viruses were shed into feces for 8 days post-infection. No recombinant viruses could be detected in nasal swabs at 0, 1, or 2 weeks post-inoculation. Anti-HIV IgG and IgA antibody response against env and gag could be measured in nasal swabs taken from 2 of 3 chimpanzees following Ad5-recombinant booster immunization by Western blot analysis. IgG and IgA anti-env and/or anti-gag antibodies were detected in saliva samples collected from 2 of 3 chimpanzees. Anti-env and -gag antibodies of the IgG class were detected in vaginal swabs taken from the single female chimpanzee.

Several samples which contained the greatest amount of anti-HIV antibodies of the IgA class were examined for the presence of secretory component. This was accomplished by substitution of polyclonal anti-secretory component (human) for polyclonal anti-IgA (human) in the HIV Western blot assay. Secretory anti-HIV IgA, containing secretory component, was detected in both nasal swabs and saliva samples in 1 of 3 chimpanzees.

**3rd Booster Immunization with env Subunit:** The strongest anti-env antibody responses were measured following subunit administration of these chimpanzees that had been primed with live recombinant adenoviruses. Anti-env antibody responses were detected in both serum and in various secretory samples collected from the nasal-oral cavity, vagina, and rectum. Peak antibody titers were detected at 4 weeks post administration with env subunit.

Serum anti-HIV neutralizing antibody titers of 320-640 were observed in all 3 chimpanzees. Antibodies directed against the gp120 V3 loop were detected by ELISA and against the gp120 CD4 binding site were detected by a FACS blocking assay. All three chimpanzees produced high ELISA titers (1000 - 9000) directed at the V3 loop (a region which contains the major neutralization determinant for HIV).

Chimpanzee sera collected at the height of the neutralizing response was evaluated for the presence of anti-CD4 binding site antibodies. All three animals had acquired antibodies that were capable of blocking the interaction between gp120 with CD4. The CD4 binding site is a conformational epitope and antibodies directed at this site are believed to be important in blocking uptake up cell-free HIV and perhaps capable of inhibiting gp120-CD4 syncytium induction. The results are shown in Figure 1.

Nasal swab anti-env antibody titers of the IgG and IgA classes of immunoglobulins were boosted in 3 of 3 and 2 of 3 chimpanzees, respectively, following booster immunization with the env subunit. Similar results were observed in the saliva samples taken from these chimpanzees. Two of three chimpanzees had IgG anti-env antibodies present in rectal swabs and the single female chimpanzee had a strong IgG anti-env booster response measured in vaginal swabs. The presence of anti-HIV antibodies in mucosal secretions is critical because certain mucosal surfaces represent major sites for HIV infection.

**Summary Tables:** The following table shows the results obtained using treatment regimen 3.

| **RESULTS OBTAINED USING TREATMENT REGIMEN 3** | | | | | | |
|---|---|---|---|---|---|---|
| Chimp Number | Recombinant Virus | Recombinant Virus Shedding Stools (Days) | Peak Anti-Adeno Neutralizing Titer | Western Blot Peak anti-HIV Titers | | Peak Anti-HIV Neutralizing Titer |
| | | | | env | gag | |
| 4 | Ad7-env, Ad7-gag | 22,22 | 1024 | 100 | 1000 | <10 |
| | Ad4-env, Ad4-gag | 14,14 | 128 | 10000 | 10000 | <10 |
| | Ad5-env, Ad5-gag | 8,8 | 32 | 10000 | 10000 | 20 |
| | subunit: env | N/A* | N/A | >10000 | 10000 | 640 |
| | | | | | | |
| 5 | Ad7-env, Ad7-gag | 34.27 | 1024 | 100 | 10000 | <10 |
| | Ad4-env, Ad4-gag | 14,14 | 512 | 10000 | 10000 | <10 |
| | Ad5-env. Ad5-gag | 8,8 | 32 | 10000 | 10000 | 20 |
| | subunit: env | N/A | N/A | 1000 | 10000 | 320 |
| | | | | | | |
| 6 | Ad7-env | 34 | 1024 | 100 | N/A | <10 |
| | Ad4-env | 28 | 512 | 10000 | N/A | <10 |
| | Ad5-env, gag | 8,8 | 32 | 10000 | N/A | 40 |
| | subunit: env | N/A | N/A | >10000 | N/A | 320 |

| | | | | | | |
|---|---|---|---|---|---|---|
| *N/A = not applicable. | | | | | | |

The following table summarizes anti-HIV responses detected in chimpanzee secretions following intranasal booster immunization with the Ad5-HIV recombinants and after the intramuscular subunit boost (week 23 post boost).

| **ANTI-HIV RESPONSES DETECTED IN SECRETIONS** | | | | | | | | |
|---|---|---|---|---|---|---|---|---|
| | | | Secretion Analyzed | | | | | |
| Chimp Number | Antigen Recognized | Weeks Post Boost** | Nasal | | Saliva | | Vaginal | |
| | | | IgA | IgG | IgA | IgG | IgA | IgG |
| 4 | env | 0 | -* | 360 | - | - | - | - |
| | | 1 | 180 | 360 | - | - | - | 90 |
| | | 2 | 180 | 2880 | 20 | 20 | - | 90 |
| | | 4 | 720 | 1440 | - | 20 | - | 360 |
| | | 23 | - | - | - | 80 | - | - |
| | | 24 | 90 | 720 | - | - | - | - |
| | | 25 | 90 | 2880 | 20 | 160 | - | 180 |
| | | 27 | 90 | 1440 | - | 160 | - | 720 |
| | | | | | | | | |
| | gag | 0 | - | 180 | - | - | - | - |
| | | 1 | 360 | 360 | - | 20 | - | 90 |
| | | 2 | 720 | 2880 | - | 20 | - | 90 |
| | | 4 | 720 | 720 | - | 20 | - | 90 |
| | | 23 | 90 | - | - | - | - | - |
| | | 24 | 90 | 90 | - | - | - | - |
| | | 25 | - | 90 | - | - | - | - |
| | | 27 | 90 | 360 | - | - | - | - |
| | | | | | | | | |
| 5 | env | 0 | - | - | - | - | N/A⁺ | N/A |
| | | 1 | - | 90 | - | - | N/A | N/A |
| | | 2 | - | 2880 | - | - | N/A | N/A |
| | | 4 | - | 360 | - | - | N/A | N/A |
| | | 23 | - | - | - | - | N/A | N/A |
| | | 24 | - | 360 | - | 20 | N/A | N/A |
| | | 25 | 90 | 2880 | 20 | 80 | N/A | N/A |
| | | 27 | - | 720 | - | 320 | N/A | N/A |
| | | | | | | | | |
| | gag | 0 | - | - | - | - | N/A | N/A |
| | | 1 | - | 90 | - | - | N/A | N/A |
| | | 2 | 90 | 1440 | - | - | N/A | N/A |
| | | 4 | - | 360 | - | - | N/A | N/A |
| | | 23 | 90 | - | - | - | N/A | N/A |
| | | 24 | 90 | - | - | - | N/A | N/A |
| | | 25 | 90 | 90 | - | - | N/A | N/A |
| | | 27 | - | - | - | - | N/A | N/A |
| | | | | | | | | |
| 6 | env | 0 | - | - | - | - | N/A | N/A |
| | | 1 | - | - | - | - | N/A | N/A |
| | | 2 | - | 1440 | 20 | 20 | N/A | N/A |
| | | 4 | - | 360 | - | - | N/A | N/A |
| | | 23 | - | - | - | - | N/A | N/A |
| | | 24 | - | 180 | - | - | N/A | N/A |
| | | 25 | - | 720 | - | 20 | N/A | N/A |
| | | 27 | - | 720 | - | - | N/A | N/A |

| | | | | | | | | |
|---|---|---|---|---|---|---|---|---|
| * - equals less than 90 for nasal and vaginal swabs and less than 20 for saliva samples. | | | | | | | | |
| + N/A = not applicable. | | | | | | | | |
| ** Post Ad5 -boost. Subunit boost was administered 23 weeks after Ad5 boost. | | | | | | | | |

### Measurement of Immunogenicity: Treatment Regimen 4

### Dog Inoculations and Collection of Data

Recombinant adenovirus was administered according to the table shown above for Treatment Regimen 4. Serum was collected at different times during the course of the experiment, and processed as described in Regimen 1. Adenovirus neutralization test procedures were performed according to the procedures described in Regimen 1. Detection of anti-HIV antibodies was performed according to the procedure described in Regimen 1 except that biotinolylated goat anti-dog IgG_{(H + L)} was substituted for biotinylated goat anti-human IgG_{(H + L)}.

Serum samples were taken from immunized dogs at regular intervals after primary immunization and booster immunizations. Seroconversion to the serotype of the adenovirus vector employed was determined by neutralization test procedures. All of the dogs responded with strong anti-adenovirus titer to Ad7 vectors. Weaker anti-Ad5 responses were seen following Ad5 primary or booster inoculation. Seroconversion to env antigens was measured by Western blot and by HIV neutralization assays. Some dogs were able to produce low titer anti-env antibodies following primary immunization with recombinant Ad-env (LAV or MN). Significant booster responses to env antigen were observed in almost all of the dogs following heterotypic boosting with another recombinant Ad-env (LAV or MN) virus expressing the same type of env antigen.

Dogs that were primed with Ad7-env_{MN} and boosted with Ad5-env_{MN} had an average anti-HIV_{MN} serum titer of >180 (range 45->270) at 4 weeks post-boost. Dogs receiving the Ad5-env_{MN} and Ad4-env_{MN} combination had an average anti-HIV_{MN} serum titer of >170 (range 45->270) at this same time. There were no cross protective antibodies directed at the HIV_{LAV} strain in any of these dogs. Dogs receiving the Ad7-env and Ad5-env combination had an average anti-HIV_{LAV} serum titer of 55 (range 20-85) at 4 weeks post-boost and none of these dogs had anti-HIV_{MN} titers. In at least one of the three dogs receiving the "recombinant cocktail" that contained both MN and LAV recombinant viruses had a anti-HIV_{MN} serum titer of 90 and an anti-HIV_{LAV} titer of 50. The other two dogs had anti-HIV_{LAV} titers of 45 and 15.

These results demonstrate that the recombinant Ad-HIV_{MN} viruses all elicit neutralizing antibodies directed at the MN strain of HIV. Low neutralizing titers were seen in 2 of 6 dogs in Groups 1 and 1 of 6 in Group 4 following the first immunization with Ad-env_{MN} recombinants. Low to high neutralization titers were measured in all of the dogs in these two groups following booster immunization with heterotypic recombinant viruses. The neutralization titers produced were type specific and did not cross react with the LAV strain of HIV. When compared directly to other dogs treated with LAV recombinant Ad-env viruses, Ad-env_{MN} recombinant viruses appeared to elicit higher type-specific neutralization titers in the dog standard pharmacological test procedure. Finally, the use of a "recombinant cocktail" which contains both MN and LAV recombinants appears to be capable of eliciting neutralizing antibodies to both strains of HIV.

### Measurement of Immunogenicity: Treatment Regimen 5 HIV Subunit Administration in Dogs

Thirty laboratory dogs that were either previously immunized twice with Ad-env recombinants (12-18 week intervals, with the 2nd immunization 20-28 weeks prior to the 1st subunit immunization) and ten (10) control dogs that have never been exposed to Ad-env recombinants were injected with one of five different HIV-env subunit preparations according to the description shown above for Treatment Regimen 5. All immunizations were administered by the subcutaneous route. Serum was collected at different times during the course of the experiment, and processed as described in Regimen 1. Adenovirus neutralization test procedures were performed according to the procedures described in Regimen 1.

### Results

The results that were obtained are described below and provided in a summary table that follows.

1st Subunit Administration. All subunit vaccines administered to Ad-env "primed" dogs boosted anti-HIV_{MN} neutralizing antibody responses. Two subunit preparations, A and C, were both examined for their ability to induce cross neutralizing responses to HIV_{SF2}. Heterologous boosting (i.e., Ad-env_{MN} primed and gp-120_{SF2} boost) as well as homologous boosting (Ad-env_{MN} primed and gp160_{MN} boost) both stimulated anti-HIV_{SF2} neutralizing antibody responses. Control dogs from groups B and C produced anti-env binding antibodies to HIV-env. Neutralizing antibody responses were not observed in control dogs following the first subunit administration.

2nd Subunit Administration. Administration of the second subunit did not appear to be as effective as a boosting agent compared to the first subunit administration. Group B dogs exhibited the greatest serum neutralizing antibody response (3-4 fold increase) of the five groups following the second booster immunization. Groups A and C showed two-fold increases following their second subunit administrations, while the HA-env antigen failed to significantly alter the geometric mean neutralizing titer of either Group D or E. Controls from all five groups produced anti-env binding antibodies. Functional neutralizing anti-HIV antibodies were observed only in the groups B, C, and D controls. Group A and E controls still failed to produce neutralizing antibody responses after the second subunit administration.

In summary, these results demonstrate that strong neutralizing antibody responses were elicited in all groups that were previously "primed" with Ad-HIV recombinants. After priming, high neutralizing antibody titers were observed in groups that were boosted heterologously (with gp120_{SF2}) and homologously (with gp120_{MN}). In the primed dogs, neutralizing antibodies were generated to both the MN and SF2 strains of HIV. Neutralizing antibody titers were still observed at twelve weeks, prior to the second boost. After the second boost, significant increases in neutralizing antibodies were observed in both gp120-boosted groups (Groups A and B).

### Summary Table

The following table shows the results obtained using Treatment Regimen 5.

| **HIV SUBUNIT IMMUNIZATION IN Ad-HIV PRIMED DOGS** | | | | | | | | |
|---|---|---|---|---|---|---|---|---|
| Group⁺ | n | First Subunit | Second Subunit | Peak Titer After 2nd Ad-HIV | Anti-HIV_{MN} Responses* | | | |
| | | | | | 0 wk | 2 wk | 12 wk | 14 wk |
| A | 6 | gp120_{SF2} | gp120_{SF2} | 122 | 16 | 357 | 84 | 270 |
| | 2 | gp120_{SF2} | gp120_{SF2} | - | - | - | - | - |
| | | | | | | | | |
| B | 6 | gp120_{MN} | gp120_{MN} | 141 | 17 | 883 | 229 | 472 |
| | 2 | gp120_{MN} | gp120_{MN} | - | - | - | - | 156 |
| | | | | | | | | |
| C | 6 | gp160_{MN} | gp160_{MN} | 88 | 29 | 369 | 55 | 68 |
| | 2 | gp160_{MN} | gp160_{MN} | - | - | - | - | 100 |
| | | | | | | | | |
| D | 6 | gp160_{MN} | HA-env | 88 | 25 | 391 | 87 | 83 |
| | 2 | gp160_{MN} | HA-env | - | - | | - | 93 |
| | | | | | | | | |
| E | 6 | HA-env | HA-env | 189 | 41 | 431 | 62 | 110 |
| | 2 | HA-env | HA-env | - | - | - | - | - |

| | | | | | | | | |
|---|---|---|---|---|---|---|---|---|
| ⁺ Each group consisted of 6 dogs that were previously immunized twice with Ad-Env_{MN} and 2 control dogs that were not immunized. | | | | | | | | |
| * Reciprocal geometric mean neutralization titer to HIV_{MN}. Reciprocal geometric mean neutralization titers to of 98 and 42 to HIV_{SF2} were observed for the previously immunized dogs of groups A and C respectively, at 2 weeks. | | | | | | | | |

### Measurement of Immunogenicity: Treatment Regimen 6 Chimpanzee Inoculations and Collection of Data

Six female chimpanzees were selected on the basis of their serological profiles to human adenoviruses types 4,5, and 7, and were treated according to the table shown above for Treatment Regimen 6. Their selection was based on a "best fit" for having the lowest possible serum neutralization titers directed at the various Ad-env vaccine combinations that were designated to be administered. Four chimpanzees that were either seronegative or weakly seropositive received either 1, 2, or 3 consecutive intranasal immunizations with recombinant Ad-env (12 week intervals) (Chimpanzees 7, 8,9, and 11). One chimpanzee that was strongly seropositive (titers of 128 to all 3 Ad serotypes; Chimpanzee 10) was given a mixture of all 3 recombinants (each at a dose of 1 x 10⁷ pfu) as a primary immunization and boosted 12 weeks later with the same mixture. All of the Ad-env immunized chimpanzees received an intramuscular immunization boost with 50 µg of gp120_{SF2} HIV-env subunit formulated in MF59 adjuvant (MF59 adjuvant is described in Vaccine 11: 909 (1993)). One control chimpanzee (number 12) received 3 consecutive intranasal immunizations with wild-type human adenoviruses (12 week intervals) and an intramuscular immunization with the MF59 adjuvant alone at week 48. Antibiotics were administered prophylactically to all of the chimpanzees and no respiratory disorders were observed.

Whole blood, serum, and stool samples were collected at different times during the course of the experiment, and processed as described in Regimen 1. Adenovirus detection in stool samples, nasal or pharyngeal swab samples were done either by a plaque hybridization assay (described in Regimen 1) or by PCR technology (see below). Adenovirus neutralization assays and detection of anti-HIV antibodies were performed according to the procedures described in Regimen 1. Chinese hamster ovary cell (CHO)-derived gp120 or commercially purchased (American Biotechnologies, Cambridge, MA) HIV V3_{MN} peptides were used as substitute antigen reagents in antibody binding assays.

PCR detection of recombinant Ad-env in chimpanzee stool samples was carried out with a commercially purchased PCR kit according to the supplier's instructions (Perkin Elmer Cetus, Norwalk CT). Briefly, about 250 µl of the stool samples was heated to 95° C for 5 minutes and centrifuged in a microfuge at top speed for 2-3 minutes. The supernatant was saved. 1-10 µl per PCR reaction was used. Several tubes of master mix were prepared from the PCR kit and kept frozen at -20° C. For a 10 reaction tube, sterile water (615 µl), 10 X buffer (100µl), dATP (20 µl), dCTP (20 µl), dGTP (20 µl), and dTTP (20 µl) were mixed to make up the master mix. For each reaction, 79.5 µl of the master mix were used. On the day of the first PCR, a tube of master mix (10 rx) was thawed. To the master mix were added 10 µl of each of the oligomers, 5 µl of native Taq DNA polymerase, 50 µl water. The solution was mixed and about 90 µl was distributed to each reaction tube. The PCR was carried out in a 0.5 ml eppendorf tube. To each tube was added 10 µl of the stool supernatant. Thirty (30) cycles of PCR amplification were run at 95° C for 1 hour, 45° C for 1.5 hours, and 72° C for 2 hours. A second PCR was performed with a 2.5 µl aliquot of the first PCR product as a DNA template and a corresponding oligo pair as primers. After 30 cycles of amplification, 10µl of the reaction product was run on a 1.2% argose gel. A 800 bp DNA band was observed as a positive control for Ad7-env. The following primer pairs were used for nested PCR.

| Template Gene | 1st PCR | 2nd PCR | DNA Size |
|---|---|---|---|
| HIV-1 gp120_{MN} | 5166/5209 | 5164/5208 | 800 bp |
| Ad4 fiber | 5467/5468 | 5469/5470 | 782 bp |
| Ad5 fiber | 5625/5523 | 5624/5522 | 423 bp |
| Ad7 fiber | 5505/5504 | 5503/5502 | 978 bp |

HIV specific CTL activity was measured by determining lysis of syngenic target cells that were infected with vaccinia virus recombinants that express either the HIV-env gene products, the HIV-gag gene products, or the lac gene product (control for nonspecific cytotoxicity).

### Results

1st Immunization with Ad5-recombinants: Recombinant Ad5 virus was shed into fecal, pharyngeal, and/or nasal specimens for 0-7 days collected from chimpanzees that were seronegative or weakly seropositive to Ad5. Only the Ad5 recombinant was detected in the strongly seropositive chimpanzee immunized with the mixture of three recombinants. Wild-type adenovirus was shed for 56 days in the control chimpanzee that was weakly seropositive to Ad5. Significant anti-Ad5 responses were produced in most of the chimpanzees, with the strongest response produced in the control animal immunized with the wild-type Ad5. Three of the four chimpanzees (numbers 7, 9 and 11) immunized with the single Ad5 recombinant produced weak anti-env antibody responses. Functional serum neutralizing anti-HIV antibodies were detected only in chimpanzee 5, which was originally seronegative to Ad5. Secretory anti-IgG anti-env antibodies were detected in vaginal, nasal, and saliva specimens collected from chimpanzee 11. Sporadic detection of env-specific CTL activity (specific lysis ≥10%) was observed in *in vitro* stimulated peripheral blood lymphocyte (PBL) populations obtained from chimpanzees 7, 8, 9, and 10 following the primary immunization with Ad5-env. Significant CTL responses were not observed in PBL obtained from chimpanzee 11.

2nd Immunization with Ad7-recombinants. Recombinant Ad7 viruses were shed into fecal, pharyngeal, and/or nasal specimens for 7-10 days in the three chimpanzees (numbers 8, 9, and 11) that were immunized with the Ad7-env alone and for 7 days in the chimpanzee (number 10) that was strongly seropositive to all 3 recombinant adenoviruses. Wild-type Ad7 was shed for 14 days in the control chimpanzee (number 12). Significant anti-Ad7 responses were developed in all Ad7 immunized animals with the best response observed in the control chimpanzee immunized with wild-type virus. Significant anti-env responses were boosted in 2 (numbers 9 and 11) of the 3 chimpanzees boosted with Ad7-env alone, while insignificant changes were observed in the animal given the mixed adenovirus preparation. Importantly, the two chimpanzees both contained functional neutralizing antibodies to HIV_{MN}. Chimpanzee 11 also had a very low cross-negative neutralizing antibody response directed at HIV_{SF2}. Nasal and saliva specimens collected from this chimpanzee also became positive for anti-env IgG antibodies. Vaginal anti-env IgG antibody responses were also boosted in chimpanzee 11. Still, anti-env antibody responses were not observed in any of the secretory fluids collected from the other chimpanzees. Again, only sporadic detection of env-specific CTL responses were detected in *in vitro* stimulated PBL populations prepared from chimpanzees 8, 9, and 10. As before, significant CTL responses were not observed in PBL populations obtained from chimpanzee 11.

3rd Immunization with Ad4-env recombinants. Recombinant Ad4-env was shed in stools for up to 3 days in the single animal (number 11) that was immunized with the Ad4-env alone. Ad4-env shedding was not detected in the strongly anti-Ad seropositive chimpanzee (number 10) after either immunization with the mixed Ad-env preparation. Wild-type Ad4 was shed for 7 days in chimpanzee 12. Both chimpanzees 11 and 12 made excellent anti-Ad4 antibody responses. The second booster immunization in chimpanzee 11 resulted with a significant boost in the anti-env antibody responses, including anti-HIV_{MN} neutralizing antibody response. Nasal, vaginal, and saliva anti-env IgG antibody responses were boosted in samples collected from chimpanzee 11. Despite the generation of an excellent humoral anti-env immune response in chimpanzee 11, significant CTL responses were not observed.

1st Subunit boost. The heterologous gp120_{SF2} subunit antigen preparation was administered to chimpanzee 7, 26 weeks after the primary Ad5-env immunization. The subunit immunization was very successful in boosting the anti-env antibody response. A high titered neutralizing anti-HIV_{MN} response (>400)was observed along with a lower anti-HIV_{SF2} response (100). The subunit administration also elicited strong anti-env IgG antibody responses in nasal and vaginal secretions, as well as weaker anti-env responses in rectal secretions. One (chimpanzee 9) of the two animals given the (Ad5-env)/(Ad7-env) combination also showed excellent anti-env booster antibody responses following subunit administration. This animal had similar anti-HIV_{MN} and anti-HIV_{SF2} neutralizing titers as seen in chimpanzee 1. Weak anti-env IgG responses were observed in nasal, rectal, saliva, and vaginal secretions collected from this animal. The other chimpanzee (number 8) had a much weaker, but still significant anti-env response induced following subunit administration, but this response did not include functional neutralizing antibodies to HIV. Nor were anti-env antibodies detected in any of the secretions collected from this chimpanzee. Chimpanzee 11 which received the (Ad5-env)/(Ad7-env)/(Ad4-env) combination also showed an excellent anti-env antibody booster response. This included a very high neutralization titer (>1400) to HIV_{MN} and high (>400) titers to HIV_{SF2}. Excellent anti-env IgG antibody responses were observed in vaginal, nasal, and saliva specimens. A weak anti-env response was observed in pharyngeal secretions. The subunit did not have a significant effect on the anti-env antibody response (serum or secretory) of chimpanzee 10 (the strongly anti-Ad seropositive animal). Sporadic anti-env CTL activity was detected in *in vitro* stimulated PBL populations collected only from chimpanzees 7 and 8 following subunit administration. Similar analysis of in *vitro* stimulated lymph node cells (obtained from a lymph node biopsy located in close proximity to the subunit inoculation site) revealed that cells obtained only from chimpanzee 8 (basically a non-humoral responder) contained significant CTL activity directed at both env_{SF2} and env_{MN}.

2nd Subunit boost. Only chimpanzee 7 received a second subunit immunization. This second immunization resulted with a significant boost in the anti-env antibody response, including high titered anti-HIV_{MN} (>200) and low (<100) anti-HIV_{SF2} neutralizing antibody responses. Excellent anti-env IgG responses were observed in vaginal, nasal, and rectal specimens. Sporadic anti-HIV CTL activity was also seen in PBL populations.

HIV_{SF2} Challenge of Immunized and Control Chimpanzees. A cell-free HIV_{SF2} challenge was administered intravenously to five of the six chimpanzees (7, 8, 9, 11, 12). The challenge stock dilution of 1/40 was shown to productively infect control chimpanzees within 3 to 4 weeks. The chimpanzees were monitored for signs of HIV infection for a period of 10 weeks. HIV could be co-cultured from PBLs obtained from control chimpanzee 12 collected at 4 and 6 weeks post-challenge. Anti-gag antibody responses were readily measurable (another indication of HIV infection since the recombinant vaccines lacked gag determinants) in serum samples collected at 6, 8, and 10 weeks post-challenge. All other chimpanzees were protected from the HIV challenge at 10 weeks.

These results demonstrate that the intranasal administration of the Ad-env recombinants (particularly Ad7-env_{MN}, Ad5-env_{MN}, Ad4-env_{MN} or a combination thereof) elicited the production of neutralizing antibodies against HIV-1. Neutralizing antibodies were produced following the first administration of the Ad-env recombinants, and the titer was increased through the use of one or more booster intranasal immunizations with the Ad-env recombinants. Antibody response to both the MN and SF2 strains of HIV was further boosted through the administration of one or more inoculations with an env (gp120) subunit antigen preparation (particularly gp120_{SF2}). Most importantly, protection against HIV-1 infection was demonstrated following the administration of the Ad-env / subunit booster treatment regimen.

## Claims

1. Use of the live recombinant adenoviruses in which the virion structural protein is unchanged from that in the native adenovirus from which the recombinant adenovirus is produced, and which contain the env gene from the MN strain of human immunodeficiency virus type 1 to prepare a medicament for use in treatment to produce antibodies or cell mediated immunity to human immunodeficiency by virus type 1, the treatment being carried out by intranasal, intramuscular or subcutaneous administration of the said live recombinant adenovirus.

2. Use according to claim 1 wherein the live recombinant adenovirus is selected from one or more of the group of consisting of adenovirus type 4, adenovirus type 5, and adenovirus type 7, wherein a gene in the early region 3 has been deleted in the adenovirus.

3. Use according to claim 2 wherein the recombinant adenovirus is selected from one or more of the group consisting of Ad7-tplenv_{MN}-tplHrev, Ad4-tplenv_{MN}-tplHrev, and Ad5-tplenv_{MN}-tplHrev.

4. Use according to claim 3, wherein the treatment further comprises administering one or more than one human immunodeficiency type 1 subunit protein.

5. Use according to claim 4, wherein the subunit protein is env or gag.

6. Use according to claim 5, wherein the human immunodeficiency type 1 subunit protein is administered subsequent to administration of the live recombinant adenovirus.

7. Use according to claim 6, wherein the env subunit protein is gp120_{MN} or gp120_{SF2}

8. Use according to claim 3, wherein the medicament is for intranasal administration of the live recombinant adenovirus and subsequent administration of one or more doses of a human immunodeficiency type 1 subunit protein.

9. Use according to claim 8, wherein the human immunodeficiency type 1 subunit protein is env.

10. Use according to claim 9, wherein the env subunit protein is gp120_{MN} or gp120_{SF2}.

11. Use according to claim 1, in which the medicament is for intranasal administration of the live recombinant virus and subsequent administration of one or more doses of a human immunodeficiency type 1 subunit protein.

12. Use according to claim 11, wherein the live recombinant adenovirus is Ad7-tplenv-tplHrev, Ad7-tplgag-tplHrev, Ad7-rev-gag, Ad4-tplenv-tplHrev, Ad4-tplgag-tplHrev, Ad4-rev-gag, Ad5-tplenv-tplHrev, Ad5-tplgag-tplHrev, Ad7-tplenv_{MN}-tplHrev, Ad4-tplenv_{MN}-tplHrev, or Ad5-tplenv_{MN}-tplHrev, or a combination thereof.

13. Use according to claim 12, wherein the live recombinant adenovirus is Ad7-tplenv_{MN}-tplHrev, Ad4-tplenv_{MN}-tplHrev, or Ad5-tplenv_{MN}-tplHrev, or a combination thereof.

14. Use according to claim 13, wherein the human immunodeficiency type 1 subunit protein is env.

15. Use according to claim 14, wherein the env subunit protein is gp120_{MN} or gp120_{SF2}.

16. A vaccine for use in treatment to produce antibodies or cell mediated immunity to human immunodeficiency virus type 1, comprising live recombinant adenoviruses in which the virion structural protein is unchanged from that in the native adenovirus from which the recombinant adenovirus is produced, and which contain the env gene from the MN strain of human immunodeficiency virus type 1coding for the antigen corresponding to said antibodies or inducing said cell mediated immunity, said vaccine being formulated in an intranasal, intramuscular, or subcutaneous dosage form.

17. The vaccine according to claim 16 wherein the live recombinant adenovirus is selected from one or more of the group of consisting of adenovirus type 4, adenovirus type 5, and adenovirus type 7, wherein a gene in the early region 3 has been deleted in the adenovirus.

18. The vaccine according to claim 17, wherein the recombinant adenovirus is selected from one or more of the group consisting of Ad7-tplenv_{MN}-tplHrev, Ad4-tplenv_{MN}-tplHrev, and Ad5-tplenv_{MN}-tplHrcv.

19. The vaccine according to claim 18 which further comprises one or more than one human immunodeficiency type 1 subunit protein for administration in the treatment.

20. The vaccine according to claim 19, wherein the subunit protein is administered subsequent to the live recombinant adenovirus.

21. The vaccine according to claim 20, wherein the subunit protein is env or gag.

22. The vaccine according to claim 21, wherein the env subunit protein is gp120_{MN} or gp120_{SF2}.

23. A vaccine as claimed in claim 16, the vaccine being a two stage vaccine for protection against human immunodeficiency type 1 infection, the vaccine having a first stage comprising said live recombinant adenovirus, said first stage being formulated in an intranasal dosage form; and a second stage comprising a human immunodeficiency type 1 subunit protein, said second stage being formulated in an intramuscular dosage form; said first stage being for administration prior to the administration of said second stage.

24. The vaccine of claim 23, wherein the live recombinant adenovirus is Ad7-tplenV_{MN}-tplHrev, Ad4-tplenv_{MN}-tplHrev, or Ad5-tplenv_{MN}-tplHrev, or a combination thereof.

25. The vaccine of claim 24, wherein the human immunodeficiency type 1 subunit protein is env.

26. The vaccine of claim 25, wherein the env subunit protein is gp120_{MN} or gP120_{SF2}.

27. A recombinant adenovirus which is X-tplY-tplHrev; wherein X is Ad4, Ad5, or Ad7; Y is env; and the env is from the MN strain of HIV-1.

28. The recombinant adenovirus according to claim 27 which is Ad7-tplenv_{MN}-tplHrev.

29. The recombinant adenovirus according to claim 27 which is Ad4-tplenv_{MN}-tplHrev.

30. The recombinant adenovirus according to claim 27 which is Ad5-tplenv_{MN}-tplHrev.

## Patentansprüche

1. Verwendung der lebenden Rekombinant-Adenoviren, in welchen das Virion-Strukturprotein unverändert gegenüber demjenigen im natürlichen Adenovirus ist, aus welchem der Rekombinant-Adenovirus hergestellt wird, und welche das env-Gen aus dem MN-Stamm vom humanen Immundefektvirus Typ 1 enthalten, um ein Medikament zur Verwendung bei Behandlung zur Erzeugung von Antikörper oder zellvermittelte Immunität gegenüber humanem Immundefekt durch Virus Typ 1 herzustellen, wobei die Behandlung durch intranasale, intramuskuläre oder subkutane Verabreichung des lebenden Rekombinant-Adenovirus durchgeführt wird.

2. Verwendung gemäß Anspruch 1, wobei das lebende Rekombinant-Adenovirus ausgewählt wird aus einem oder mehreren der Gruppe bestehend aus Adenovirus Typ 4, Adenovirus Typ 5 und Adenovirus Typ 7, wobei ein Gen in der frühen Region 3 im Adenovirus entfernt worden ist.

3. Verwendung gemäß Anspruch 2, wobei das Rekombinant-Adenovirus ausgewählt wird aus einem oder mehreren der Gruppe bestehend aus Ad7-tplenv_{MN}-tplHrev, Ad4-tplenv_{MN}-tplHrev und Ad5-tplenv_{MN}tplHrev.

4. Verwendung gemäß Anspruch 3, wobei die Behandlung ferner Verabreichen von einem oder mehr als einem humanen Immundefekt Typ 1 Subunit-Protein umfasst.

5. Verwendung gemäß Anspruch 4, wobei das Subunit-Protein env oder gag ist.

6. Verwendung gemäß Anspruch 5, wobei das humane Immundefekt Typ 1 Subunit-Protein nach Verabreichung des lebenden Rekombinant-Adenovirus verabreicht wird.

7. Verwendung gemäß Anspruch 6, wobei das env-Subunit-Protein gp120_{MN} oder pg120_{SF2} ist.

8. Verwendung gemäß Anspruch 3, wobei das Medikament zur intranasalen Verabreichung des lebenden Rekombinant-Adenovirus und nachfolgenden Verabreichung von einer oder mehrerer Dosen von einem humanen Immundefekt Typ 1 Subunit-Protein bestimmt ist.

9. Verwendung gemäß Anspruch 8, wobei das humane Immundefekt Typ 1 Subunit-Protein env ist.

10. Verwendung gemäß Anspruch 9, wobei das env-Subunit-Protein gp120_{MN} oder ph120_{SF2} ist.

11. Verwendung gemäß Anspruch 1, wobei das Medikament zur intranasalen Verabreichung des lebenden Rekombinant-Virus und nachfolgenden Verabreichung von einer oder mehrerer Dosen von einem humanen Immundefekt Typ 1 Subunit-Protein bestimmt ist.

12. Verwendung gemäß Anspruch 11, wobei das lebende Rekombinant-Adenovirus Ad7-tplenv-tplHrev, Ad7-tplgag-tplHrev, Ad7-revgag, Ad4-tplenv-tplHrev, Ad4-tplgag-tplHrev, Ad4-rev-gag, Ad5-tplenv-tplHrev, Ad5-tplgag-tplHrev, Ad7-tplenv_{MN}-tplHrev, Ad4-tplenv_{MN}-tplHrev oder Ad5-tplenv_{MN}-tplHrev oder eine Kombination davon ist.

13. Verwendung gemäß Anspruch 12, wobei das lebende Rekombinant-Adenovirus Ad7-tplenv_{MN}-tplHrev, Ad4-tplenv_{MN}-tplHrev oder Ad5-tplenv_{MN}-tplHrev oder eine Kombination davon ist.

14. Verwendung gemäß Anspruch 13, wobei das humane Immundefekt Typ 1 Subunit-Protein env ist.

15. Verwendung gemäß Anspruch 14, wobei das env-Subunit-Protein gp120_{MN} oder gp120_{SF2} ist.

16. Impfstoff zur Verwendung bei der Behandlung zum Erzeugen von Antikörpern oder zellvermittelter Immunität gegenüber humanem Immundefektvirus Typ 1, welcher lebende Rekombinant-Adenoviren umfasst, in welchen das Virion-Strukturprotein unverändert gegenüber demjenigen im natürlichen Adenovirus ist, aus welchem der Rekombinant-Adenovirus hergestellt wird, und welche das env-Gen aus dem MN-Stamm von humanem Immundefektvirus Typ 1 enthalten, kodierend für das Antigen entsprechend den besagten Antikörpern oder besagte zellvermittelte Immunität herbeiführend, wobei besagter Impfstoff in einer intranasalen, intramuskulären oder subkutanen Dosierungsform formuliert ist.

17. Impfstoff gemäß Anspruch 16, wobei das lebende Rekombinant-Adenovirus ausgewählt wird aus einem oder mehreren der Gruppe bestehend aus Adenovirus Typ 4, Adenovirus Typ 5 und Adenovirus Typ 7, wobei ein Gen in der frühen Region 3 im Adenovirus entfernt worden ist.

18. Impfstoff gemäß Anspruch 17, wobei das Rekombinant-Adenovirus ausgewählt wird aus einem oder mehreren der Gruppe bestehend aus Ad7-tplenv_{MN}-tplHrev, Ad4-tplenv_{MN}-tplHrev und Ad5-tplenv_{MN}tplHrev.

19. Impfstoff gemäß Anspruch 18, welcher ferner ein oder mehr als ein humanes Immundefekt Typ 1 Subunit-Protein zur Verabreichung bei der Behandlung umfasst.

20. Impfstoff gemäß Anspruch 19 wobei das Subunit-Protein nach dem lebenden Rekombinant-Adenovirus verabreicht wird.

21. Impfstoff gemäß Anspruch 20, wobei das Subunit-Protein env oder gag ist.

22. Impfstoff gemäß Anspruch 21, wobei das env-Subunit-Protein gp120_{MN} oder gp120_{SF2} ist.

23. Impfstoff wie in Anspruch 16 beansprucht, wobei der Impfstoff ein Zweistufenimpfstoff zum Schutz gegen humane Immundefekt Typ 1 Infektion ist, der Impfstoff eine erste Stufe hat, welche das lebende Rekombinant-Adenovirus umfasst, wobei die erste Stufe in einer intranasalen Dosierungsform formuliert ist; und eine zweite Stufe, welche ein humanes Immundefekt Typ 1 Subunit-Protein umfasst, wobei die zweite Stufe in einer intramuskulären Dosierungsform formuliert ist; wobei die erste Stufe zur Verabreichung vor der Verabreichung der zweiten Stufe bestimmt ist.

24. Impfstoff nach Anspruch 23, worin das lebende Rekombinant-Adenovirus Ad7-tplenv_{MN}-tplHrev, Ad4-tplenv_{MN}-tplHrev oder Ad5-tplenv_{MN}-tplHrev oder eine Kombination daraus ist.

25. Impfstoff nach Anspruch 24, worin das humane Immundefekt Typ 1 Subunit-Protein env ist.

26. Impfstoff nach Anspruch 25, worin das env-Subunit-Protein pg120_{MN} oder gp120_{SF2} ist.

27. Rekombinant-Adenovirus, welches X-tplY-tplHrev ist; worin X für Ad4, Ad5 oder Ad7 steht; Y env darstellt; und das env vom MN-Stamm von HIV-1 ist.

28. Rekombinant-Adenovirus gemäß Anspruch 27, welches Ad7-plenv_{MN}-tplHrev ist.

29. Rekombinant-Adenovirus gemäß Anspruch 27, welches Ad4-tplenv_{MN}-tplHrev ist.

30. Rekombinant-Adenovirus gemäß Anspruch 27, welches Ad5-tplenv_{MN}-tplHrev ist.

## Revendications

1. Utilisation d'adénovirus recombinants vivants dans lesquels la protéine structurelle du virion n'est pas modifiée par rapport à celle de l'adénovirus natif à partir duquel l'adénovirus recombinant est produit, et qui contiennent le gène env de la souche MN du virus de type 1 de l'immunodéficience humaine pour préparer un médicament destiné à une utilisation dans un traitement pour produire une immunité due à des anticorps ou cellulaire contre l'immunodéficience humaine due au virus de type 1, le traitement étant réalisé par une administration intranasale, intramusculaire ou sous-cutanée dudit adénovirus recombinant vivant.

2. Utilisation suivant la revendication 1, dans laquelle l'adénovirus recombinant vivant est sélectionné parmi un ou plusieurs parmi le groupe comprenant l'adénovirus de type 4, l'adénovirus de type 5, et l'adénovirus de type 7, dans lesquels un gène de la région précoce 3 a été délété dans l'adénovirus.

3. Utilisation suivant la revendication 2, dans laquelle l'adénovirus recombinant est sélectionné parmi un ou plusieurs parmi le groupe comprenant l'Ad7-tplenv_{MN}-tplHrev, l'Ad4-tplenv_{MN}-tplHrev et l'Ad5-tplenv_{MN}-tplHrev,

4. Utilisation suivant la revendication 3, dans laquelle le traitement comprend en outre l'administration d'une ou de plus d'une sous-unité protéique du virus de type 1 de l'immunodéficience humaine.

5. Utilisation suivant la revendication 4, dans laquelle la sous-unité protéique est env ou gag.

6. Utilisation suivant la revendication 5, dans laquelle la sous-unité protéique du virus de type 1 de l'immunodéficience humaine est administrée après l'administration de l'adénovirus recombinant vivant.

7. Utilisation suivant la revendication 6, dans laquelle la sous-unité protéique env est gp120_{MN} ou gp120_{SF2}.

8. Utilisation suivant la revendication 3, dans laquelle le médicament est destiné à une administration intranasale de l'adénovirus recombinant vivant et à une administration ultérieure d'une ou plusieurs doses d'une sous-unité protéique du virus de type 1 de l'immunodéficience humaine.

9. Utilisation suivant la revendication 8, dans laquelle la sous-unité protéique du virus de type 1 de l'immunodéficience humaine est env.

10. Utilisation suivant la revendication 9, dans laquelle la sous-unité protéique env est gp120_{MN} ou gp120_{SF2}.

11. Utilisation suivant la revendication 1, dans laquelle le médicament est destiné à une administration intranasale du virus recombinant vivant et à une administration ultérieure d'une ou plusieurs doses d'une sous-unité protéique du virus de type 1 de l'immunodéficience humaine.

12. Utilisation suivant la revendication 11, dans laquelle l'adénovirus recombinant vivant est l'Ad7-tplenv-tplHrev, l'Ad7-tplgag-tplHrev, l'Ad7-rev-gag, l'Ad4-tplenv-tplHrev, l'Ad4-tplgag-tplHrev, l'Ad4-rev-gag, l'Ad5-tplenv-tplHrev, l'Ad5-tplgag-tplHrev, l'Ad7-tplenv_{MN}-tplHrev, l'Ad4-tplenv_{MN}-tplHrev, ou l'Ad5-tplenv_{MN}-tplHrev, ou une combinaison de ceux-ci.

13. Utilisation suivant la revendication 12, dans laquelle l'adénovirus recombinant vivant est l'Ad7-tplenv_{MN}-tplHrev, l'Ad4-tplenv_{MN}-tplHrev ou l'Ad5-tplenv_{MN}-tplHrev, ou une combinaison de ceux-ci.

14. Utilisation suivant la revendication 13, dans laquelle la sous-unité protéique du virus de type 1 de l'immunodéficience humaine est env.

15. Utilisation suivant la revendication 14, dans laquelle la sous-unité protéique env est gp120_{MN} ou gp120_{SF2}.

16. Vaccin pour une utilisation dans un traitement pour produire une immunité due à des anticorps ou cellulaire contre le virus de type 1 de l'immunodéficience humaine, comprenant des adénovirus recombinants vivants dans lesquels la protéine structurelle du virion n'est pas modifiée par rapport à celle de l'adénovirus natif à partir duquel l'adénovirus recombinant est produit, et qui contiennent le gène env de la souche MN du virus de type 1 de l'immunodéficience humaine codant pour l'antigène correspondant auxdits anticorps ou induisant ladite immunité cellulaire, ledit vaccin étant formulé sous une forme posologique intranasale, intramusculaire ou sous-cutanée.

17. Vaccin suivant la revendication 16, dans lequel l'adénovirus recombinant vivant est sélectionné parmi un ou plusieurs parmi le groupe comprenant l'adénovirus de type 4, l'adénovirus de type 5, et l'adénovirus de type 7, dans lesquels un gêne dans la région précoce 3 a été délété dans l'adénovirus.

18. Vaccin suivant la revendication 17, dans lequel l'adénovirus recombinant est sélectionné parmi un ou plusieurs parmi le groupe comprenant l'Ad7-tplenv_{MN}-tplHrev, l'Ad4-tplenv_{MN}-tplHrev et l'Ad5-tplenv_{MN}-tplHrev.

19. Vaccin suivant la revendication 18, qui comprend en outre une ou plus d'une sous-unité protéique du virus de type 1 de l'immunodéficience humaine pour une administration dans le traitement.

20. Vaccin suivant la revendication 19, dans lequel la sous-unité protéique est administrée après l'adénovirus recombinant vivant.

21. Vaccin suivant la revendication 20, dans lequel la sous-unité protéique est env ou gag.

22. Vaccin suivant la revendication 21, dans lequel la sous-unité protéique est gp120_{MN} ou gp120_{SF2}.

23. Vaccin suivant la revendication 16, le vaccin étant un vaccin en deux étapes pour une protection contre une infection par le virus de type 1 de l'immunodéficience humaine, le vaccin présentant une première étape comprenant ledit adénovirus recombinant vivant, et ladite première étape étant formulée sous une forme posologique intranasale, et une deuxième étape comprenant une sous-unité protéique du virus de type 1 de l'immunodéficience humaine, ladite deuxième étape étant formulée sous une forme posologique intramusculaire; ladite première étape étant destinée à une administration avant l'administration de ladite deuxième étape.

24. Vaccin suivant la revendication 23, dans lequel l'adénovirus recombinant est l'Ad7-tplenv_{MN}-tplHrev, l'Ad4-tplenv_{MN}-tplHrev, ou l'Ad5-tplenv_{MN}-tplHrev, ou une combinaison de ceux-ci.

25. Vaccin suivant la revendication 24, dans lequel la sous-unité protéique du virus de type 1 de l'immunodéficience humaine est env.

26. Vaccin suivant la revendication 25, dans lequel la sous-unité protéique env est gp120_{MN} ou gp120_{SF2}.

27. Adénovirus recombinant qui est X-tplY-tplHrev; dans lequel X est Ad4, Ad5 ou Ad7; Y est env; et l'env provient de la souche MN du VIH-1.

28. Adénovirus recombinant suivant la revendication 27, qui est l'Ad7-tplenv_{MN}-tplHrev.

29. Adénovirus recombinant suivant la revendication 27, qui est l'Ad4-tplenv_{MN}-tplHrev.

30. Adénovirus recombinant suivant la revendication 27, qui est l'Ad5-tplenv_{MN}-tplHrev.
